# EUROPÄISCHE PATENTSCHRIFT

(11) **EP 2 682 605 B1**
(45) Veröffentlichungstag und Bekanntmachung des Hinweises auf die Patenterteilung: **06.09.2017**
(21) Anmeldenummer: 13174619.0
(22) Anmeldetag: 02.07.2013
(51) Int. Cl.: F04B 43/12, F04B 53/22, A61M 5/142

(54) **Schlauchrollenpumpe mit einem verriegelbaren Rotor und medizinisches Gerät zur extrakorporalen Blutbehandlung mit Schlauchrollenpumpe**
Hose reel pump with lockable rotor and medical device for extracorporeal blood treatment with hose reel pump
Pompe à rouleau tubulaire avec rotor verrouillable et appareil médical pour le traitement extracorporel du sang doté d'une pompe à rouleau tubulaire

(30) Priorität: 03.07.2012 DE 102012105916
(43) Veröffentlichungstag der Anmeldung: 08.01.2014
(73) Patentinhaber: B. Braun Avitum AG, 34212 Melsungen (DE)
(72) Erfinder: Schäfer, Oliver, 36286 Neuenstein (DE); Iske, Andreas, 34320 Söhrewald (DE)
(74) Vertreter: Winter, Brandl, Fürniss, Hübner, Röss, Kaiser, Polte - Partnerschaft mbB

(56) Entgegenhaltungen:
- DE-A1-102007 020 573
- DE-U1- 8 500 320
- US-A- 3 737 257
- US-A- 4 558 996
- US-A- 5 062 775

## Beschreibung

Die Erfindung betrifft eine Schlauchrollenpumpe für ein medizinisches Gerät zur extrakorporalen Blutbehandlung mit einem Pumpengehäuse, das eine gebogene Lauffläche und einen innerhalb der Lauffläche drehbaren Rotor aufweist, wobei ein Schlauchsegment zwischen die Lauffläche und den Rotor einbringbar ist. Der Rotor ist dabei auf einer Antriebswelle der Schlauchrollenpumpe anbringbar.

Die Erfindung betrifft ferner ein medizinisches Gerät zur extrakorporalen Blutbehandlung mit einer solchen Schlauchrollenpumpe.

In medizinischen Geräten zur extrakorporalen Blutbehandlung (Dialyse) werden oftmals Schlauchrollenpumpen eingesetzt, welche das entnommene Blut des Patienten zu einem Dialysator und zum Patienten zurückfördern. Solche Schlauchrollenpumpen arbeiten peristaltisch, wobei ein schlaufenförmiges Schlauchsegment an einer entsprechend gebogenen Lauffläche des Pumpengehäuses anliegt. Ein innerhalb der Lauffläche liegender Rotor der Pumpe bewegt sich dann mit seinen Außenkanten bzw. daran angebrachten Rollen entlang des Schlauchsegments, wobei es den Schlauch lokal eindrückt und so mit den elastischen Materialeigenschaften des Schlauchsegments eine Blutförderung durch das Schlauchsegment ermöglicht. Dafür wird das Blut dem Schlauchsegment über einen ersten Anschluss zugeführt und über einen weiteren Anschluss am anderen Ende des Schlauchsegments wieder abgeführt. Das Schlauchsegment bildet so beispielsweise zusammen mit den zu- und abführenden Leitungen und mehreren Luftfängern ein sogenanntes Überleitsystem, mit dem Blut des Patienten zu einem Dialysator und zum Patienten zurückgefördert wird.

Beispielsweise offenbart die Offenlegungsschrift DE 10 2007 020 573 A1 eine solche Schlauchrollenpumpe mit einem Stator, einem Rotor und einem Rotorantrieb, bei der zwischen den Rotor und die Schlauchrollenbahn des Stators ein Schlauch eingelegt wird. Durch die Rotation des Rotors und damit die Umlaufbewegung von Schlauchrollen wird der Schlauch jeweils gegen die Schlauchrollenbahn des Stators gedrückt, wodurch Flüssigkeit durch den Schlauch gepumpt wird.

Auch die Patentschrift US 7,547,200 B2 offenbart eine solche peristaltische Pumpe mit einem Rotor und Rollen, welche einen eingelegten Schlauch gegen eine halbkreisförmige Schlauchrollenbahn drücken. Dabei hat die Rollenbahn an einem Ende eine abgeschrägte Kante, um den Schlauch aufzunehmen, der an einem auswechselbaren Einsatz angebracht ist.

Die Patentschrift US 3 737 257 A offenbart ebenfalls eine peristaltische Pumpe in einem Pumpengehäuse, mit einem Rotor und Außenrollen mit einer Rollensteuerung. Über ein Verriegelungselement am Gabelkopf des Rotors lässt sich der Rotor mit der Antriebswelle in Drehrichtung koppeln. Das Verriegelungselement dient im ausgeklappten Zustand als Kurbelelement um einen Notbetrieb zu gewährleisten.

In der Patentschrift US 4 558 996 A ist eine Schlauchrollenpumpe offenbart, die einfach einzulegen ist. Sie weist ein Basiselement, einen Rotor und einen Stator auf, der Rotor sitzt auf dem Basiselement und kann über ein Verriegelungselement und einen Stift an die Antriebswelle in Drehrichtung gekoppelt werden.

Die in der Medizintechnik bei solchen Pumpen verwendeten Überleitsysteme werden üblicherweise nach jeder Behandlung ausgetauscht und nicht wieder für andere Patienten verwendet. Ein benutztes Schlauchsegment muss somit aus der Pumpe entfernt werden, bevor ein neues Überleitsystem in das Gerät eingebracht wird.

Ferner wird auch der Rotor eines solchen Systems üblicherweise nach jeder Behandlung zu Reinigungszwecken entnommen und nach der Reinigung wieder eingesetzt. Um die Handhabung des Rotors bei diesem Vorgang zu vereinfachen, sind bereits Systeme entwickelt worden, die zur Drehmomentübertragung von der Antriebswelle auf den Rotor einen radialen und axialen Formschluss verwenden. Derartig ausgestaltete Rotoren können insbesondere Bajonettverschlüsse in Kombination mit einem zusätzlichen Verriegelungselement aufweisen. Bei dem zusätzlichen Verriegelungselement kann es sich beispielsweise um einen Hebel handeln, der umgelegt werden muss, um den Rotor zu verriegeln. Jedoch sind für solche Systeme beim Einsetzvorgang wenigstens vier Handlingschritte erforderlich, denn es muss zunächst das radiale Verriegelungselement umgelegt werden, bevor der Rotor auf die Antriebswelle aufgesteckt und um etwa 90° gedreht werden kann, um den Bajonettverschluss zu verrasten. Danach muss das Verriegelungselement wieder umgelegt werden, um den Rotor abschließend zu verriegeln.

Darüber hinaus sollten derartige Verriegelungssysteme auch ein manuelles Drehen des Rotors ermöglichen, um beispielsweise bei einem Stromausfall eine manuelle Pumpfunktion gewährleisten zu können.

Aufgabe der Erfindung ist es daher, eine Schlauchrollenpumpe für ein medizinisches Gerät zur extrakorporalen Blutbehandlung mit einem Rotor bereitzustellen, die ein Einsetzen des Rotors mit möglichst wenigen und leicht durchzuführenden Handlingschritten ermöglicht, wobei insbesondere auch eine manuelle Pumpfunktion möglich sein soll.

Aufgabe der Erfindung ist es ferner, ein medizinisches Gerät zur extrakorporalen Blutbehandlung bereitzustellen, in dessen Schlauchrollenpumpe ein solcher Rotor einbringbar ist.

Erfindungsgemäß wird diese Aufgabe durch eine Schlauchrollenpumpe gemäß dem unabhängigen Anspruch 1 gelöst. Vorteilhafte Weiterbildungen der Schlauchrollenpumpe ergeben sich aus den Unteransprüchen 2-12. Die Aufgabe wird ferner durch ein medizinisches Gerät zur extrakorporalen Blutbehandlung gemäß Anspruch 13 gelöst.

Die erfindungsgemäße Schlauchrollenpumpe für ein medizinisches Gerät zur extrakorporalen Blutbehandlung weist ein Pumpengehäuse mit einer gebogenen Lauffläche und einen innerhalb der Lauffläche drehbaren Rotor auf, wobei ein Schlauchsegment zwischen die Lauffläche und den Rotor einbringbar ist. Der Rotor ist auf einer Antriebswelle der Schlauchrollenpumpe anbringbar und sowohl in axialer Richtung als auch in Drehrichtung mit der Antriebswelle (30) koppelbar.

Erfindungsgemäß ist ein an dem Rotor vorgesehenes Verriegelungselement zwischen wenigstens zwei Lagen bewegbar, insbesondere schwenkbar und die Geometrien der Antriebswelle und des Verriegelungselements so ausgeformt sind, dass der Rotor in einer ersten Lage des Verriegelungselements relativ zur Antriebswelle (40) verschiebbar und drehbar ist, während der Rotor in einer zweiten Lage des Verriegelungselements sowohl in axialer Richtung als auch in Drehrichtung, insbesondere durch Formschluss mit der Antriebswelle (40) gekoppelt bzw. an der Antriebswelle verriegelt ist.

Durch diesen Aufbau der Schlauchrollenpumpe kann der Rotor mit wenigen einfachen Handlingschritten auf der Antriebswelle sowohl axial als auch drehfest verriegelt werden, wobei mit dem schwenkbaren Verriegelungselement gleichzeitig beide Verriegelungsarten realisierbar sind. Durch die axiale Verriegelung kann der Rotor dann nicht mehr von der Antriebswelle gezogen werden, während durch die drehfeste Verriegelung eine Übertragung des Drehmoments von der Antriebswelle auf den Rotor ermöglicht wird.

Zum Einsetzen des Rotors muss das Verriegelungselement in die erste Lage bewegt oder geschwenkt werden, in welcher der Rotor ohne Verriegelung auf die Antriebswelle schiebbar ist. Anschließend wird das Verriegelungselement in die zweite Lage bewegt oder geschwenkt, wobei durch diese Bewegung gleichzeitig sowohl die axiale als auch die drehfeste Verriegelung herstellbar sind. Das System lässt sich somit mit nur einem Verriegelungselement in drei Schritten sowohl axial als auch drehfest verriegeln. Dies stellt einen Handlingvorteil gegenüber bekannten Lösungen mit mehr als drei Schritten dar. Ferner minimiert das System die Anzahl an montierten Bauteilen, was zu geringeren Teilekosten und geringeren Montagekosten führt. Außerdem lässt sich die Getriebeabtriebswelle der Pumpe, d.h. die Antriebswelle, gut reinigen.

Vorzugsweise ist das Verriegelungselement dabei innerhalb einer Nut bzw, Ausnehmung im Rotor schwenkbar, und die Verriegelungsvorgänge finden innerhalb dieser Nut statt. Der Rotor ist dann mit der Nut auf die Antriebswelle aufzusetzen, bevor die Verriegelung initiiert wird. Gegebenenfalls kann es dabei erforderlich sein, den Rotor nach dem Aufsetzen auf die Antriebswelle gegenüber dieser auszurichten, bevor das Verriegelungselement so auf die Antriebswelle umgelegt werden kann, dass die drehfeste Verriegelung durch Formschluss greifen kann.

Das Verriegelungselement und die Antriebswelle können dabei auf verschiedene Arten ausgeformt sein, um eine axiale und drehfeste Ver- und Entriegelung über einen Formschluss zu realisieren. In einem Ausführungsbeispiel der Erfindung weist das Verriegelungselement beispielsweise eine Nut bzw. Ausnehmung auf, während die Antriebswelle zwei gegenüber liegende ebene Seitenflächen aufweist, die bei einer drehfesten Verriegelung des Rotors auf der Antriebswelle an Innenflächen der Nut des Verriegelungselements anliegen. So wird der Formschluss für die drehfeste Verriegelung und die Übertragung des Drehmoments von der Antriebswelle auf den Rotor hergestellt.

Darüber hinaus kann das freie Ende der Antriebswelle oberhalb der zwei gegenüber ebenen Seitenflächen keilförmig ausgeformt sein, was das Umlegen des Verriegelungselements auf die Antriebswelle erleichtern würde, da die Nut des Verriegelungselements und damit der Rotor durch die Keilform leicht in die richtige Richtung gedreht werden, wenn die vorherige Ausrichtung des Rotors zur Antriebswelle vorher ungenau war.

Ferner kann das Verriegelungselement einen Verriegelungsabschnitt mit wenigstens einem Vorsprung mit einer nach außen gewölbten Verriegelungsfläche aufweisen, während die Antriebswelle wenigstens einen nach innen gewölbten Abschnitt aufweist, in welchen diese wenigstens eine Verriegelungsfläche des Verriegelungselements bei einer axialen Verriegelung des Rotors auf der Antriebswelle eingreift. Diese Ausgestaltung hat den Vorteil, dass der Verriegelungsabschnitt bei Drehung des Verriegelungselements gut in die Innenwölbung der Antriebswelle und wieder aus dieser heraus schwenkbar ist. Insbesondere ist dies vorteilhaft der Fall, wenn die Drehachse des Verriegelungselements durch diesen Verriegelungsabschnitt verläuft.

In einem weiteren Ausführungsbeispiel der Erfindung ist das Verriegelungselement um die Drehachse in eine dritte Lage schwenkbar, in welcher der Rotor ebenfalls durch Formschluss axial an der Antriebswelle verriegelt ist, jedoch keine drehfeste Verriegelung gegeben ist. Es lassen sich somit zwei axiale Verriegelungslagen herstellen, die unterschiedlich genutzt werden können. Insbesondere kann diese dritte Lage für eine manuelle Pumpfunktion genutzt werden, in welcher der Rotor zwar axial an der Antriebswelle verriegelt ist, jedoch kein Formschluss in Drehrichtung zwischen Antriebswelle und Rotor besteht. So kann der Rotor manuell beispielsweise mit einem Kurbelgriff gedreht werden, ohne dass er dabei axial von der Antriebswelle rutschen kann. Das Erfordernis der manuellen Pumpfunktion bei einem Stromausfall ist somit erfüllt. Der nach innen gewölbte Abschnitt der Antriebswelle läuft dabei vorzugsweise vollständig um die Antriebswelle herum, so dass die axiale Verriegelung bei manueller Drehung des Rotors erhalten bleibt.

Um den Rotor auch in dieser dritten Lage axial verriegelbar zu machen, kann der Verriegelungsabschnitt einen zweiten Vorsprung mit einer zweiten nach außen gewölbten Verriegelungsfläche aufweisen, welche in der dritten Lage des Verriegelungselements in den nach innen gewölbten Abschnitt der Antriebswelle eingreift. Die beiden nach außen gewölbten Verriegelungsflächen liegen sich vorzugsweise gegenüber und sind durch zwei gerade Seitenflächen miteinander verbunden. Diese zwei geraden Verbindungsflächen geben das System in der vollständig entriegelten, ersten Stellung axial frei. In einer Ausführungsform der Erfindung ist der Verriegelungsabschnitt dabei plattenförmig zwischen zwei Seitenteilen des Verriegelungselements ausgeformt ist.

Vorzugsweise sind die zweite und dritte Verriegelungslage durch Schwenken des Verriegelungselements aus der ersten Lage in jeweils unterschiedliche Richtungen erreichbar. Dabei weist das Verriegelungselement beispielsweise einen Kurbelgriff auf, der in der dritten Lage des Verriegelungselements aus der Außenkontur des Rotors herausragt. Diese Lage ist dann durch Schwenken des Verriegelungselements aus der ersten, entriegelten Lage in eine erste Richtung erreichbar, und durch den herausragenden Kurbelgriff kann der Bediener die manuelle Pumpfunktion durchführen.

Schwenkt man das Verriegelungselement dagegen in die andere Richtung, um über die entriegelte Lage die zweite Verriegelungslage zu erreichen, ist das gesamte Verriegelungselement in dieser zweiten Lage vorzugsweise bündig in die Außenkontur des Rotors eingelassen, so dass auch der Kurbelgriff in dem Rotor versenkt ist. In dieser Lage stören keine herausragenden Bauteile die Drehung des Rotors.

Von der Erfindung umfasst ist ferner ein medizinisches Gerät zur extrakorporalen Blutbehandlung, das eine Schlauchrollenpumpe mit einem Pumpengehäuse aufweist, welches eine gebogene Lauffläche und einen innerhalb der Lauffläche drehbaren Rotor aufweist, wobei ein Schlauchsegment eines extrakorporalen Blutkreislaufs zwischen die Lauffläche und den Rotor einbringbar ist. Die Schlauchpumpe ist dabei gemäß einer Ausführungsform der erfindungsgemäßen Schlauchpumpe ausgeführt.

Weitere Vorteile, Besonderheiten und zweckmäßige Weiterbildungen der Erfindung ergeben sich aus den Unteransprüchen und der nachfolgenden Darstellung bevorzugter Ausführungsbeispiele anhand der Abbildungen.

Von den Abbildungen zeigt:
- Fig. 1: eine schematische Darstellung eines medizinischen Geräts zur extrakorporalen Blutbehandlung mit einer Blutpumpe;
- Fig. 2: eine schematische Aufsicht auf eine Schlauchrollenpumpe mit eingelegtem Schlauchstück und Rotor;
- Fig. 3: eine schematische Seitenansicht eines Rotors bei der Montage oder Demontage;
- Fig. 4: eine schematische Seitenansicht eines Rotors im Therapiebetrieb;
- Fig. 5: eine schematische Seitenansicht eines Rotors im manuellen Notbetrieb;
- Fig. 6: eine schematische Aufsicht auf einen Rotor vor der Ausrichtung gegenüber einer Antriebswelle; und
- Fig. 7: eine schematische Aufsicht auf einen Rotor im manuellen Notbetrieb.

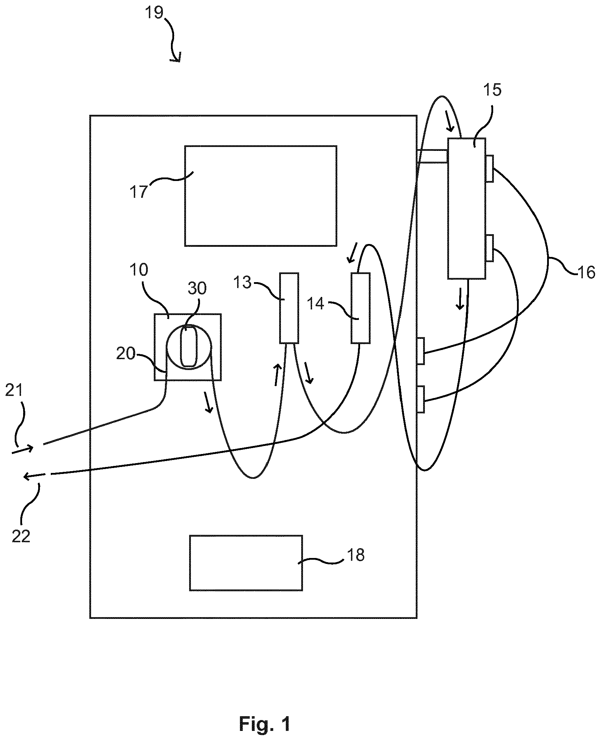

Fig. 1 zeigt eine schematische Darstellung der wesentlichen Grundkomponenten eines medizinischen Geräts 19 zur extrakorporalen Blutbehandlung mit einer Blutpumpe, wobei es sich bei der Blutpumpe um eine Schlauchrollenpumpe handelt. Die Schlauchrollenpumpe weist dabei ein Pumpengehäuse 10 auf, das typischerweise an der Frontseite des Dialysegeräts 19 angebracht ist.

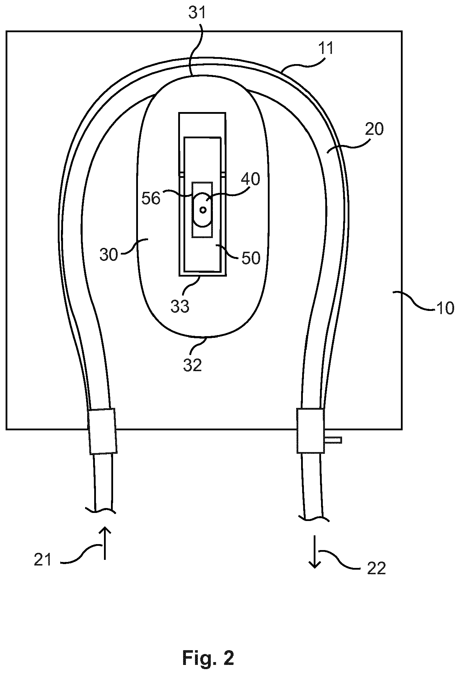

Dieser Schlauchrollenpumpe wird arterielles Blut 21 eines Patienten zugeführt und durch den extrakorporalen Blutkreislauf gefördert. Anschließend wird das Blut als venöses Blut 22 wieder zum Patienten zurückgeführt. Dabei wird das Blut mittels der Pumpe durch ein Überleitsystem gefördert, das an mehrere Komponenten des Dialysegeräts angeschlossen ist, wobei ein Schlauchsegment 20 des Überleitsystems in die Blutpumpe eingelegt ist und ein Rotor 30 das Blut peristaltisch durch dieses Schlauchsegment 20 fördert, wie es einer vergrößerten Ansicht der Fig. 2 zu entnehmen ist.

Nach Durchlaufen der Blutpumpe gelangt das Blut zum Dialysator 15, nachdem es vorzugsweise zuvor einen arteriellen Luftfänger 13 durchlaufen hat. Im Dialysator 15 wird das Blut durch Stoffaustausch mit einem Dialysat 16 gereinigt, welches dem Dialysator 15 zu- und abgeführt wird. Nach Durchlaufen des Dialysators 15 gelangt das Blut zu einem venösen Luftfänger 14 und wird anschließend dem Patienten zugeführt. Dieser Kreislauf des Bluts des Patienten ist in Fig. 1 durch Pfeile gekennzeichnet.

Die Einstellung von Parametern der Dialyse und die Überwachung der Therapie können über eine Anzeige-/Eingabeeinheit 17 erfolgen, die vorzugsweise als Touchscreen ausgebildet ist. Ferner weist das Dialysegerät 19 eine Steuer-/Regeleinheit 18 auf.

Fig. 2 stellt eine schematische Darstellung einer Aufsicht auf eine Schlauchrollenpumpe mit eingelegtem Schlauchsegment 20 und Rotor 30 dar. Die Schlauchrollenpumpe weist dabei ein Pumpengehäuse 10 auf, das für den Bediener des Geräts leicht zugänglich ist, wobei das Pumpengehäuse 10 mit einem nicht dargestellten Deckel abdeckbar ist, der über ein Scharnier zum Beispiel nach oben oder zur Seite schwenkbar ist, um Zugriff auf das Schlauchsegment 20 und den Rotor 30 zu erhalten.

In dem Pumpengehäuse 10 ist durch eine Vertiefung im Gehäuse eine kurvenförmige Lauffläche 11 ausgebildet, in welche das Schlauchsegment 20 schlaufenförmig eingelegt werden kann, so dass seine beiden Schlauchenden unten aus dem Gehäuse 10 herausragen. Dabei kann die Vertiefung mit einer Seitenfläche in dem Pumpengehäuse 10 ausgebildet sein, die im Wesentlichen gleichmäßig senkrecht zur Frontseite des Geräts verläuft, oder die Lauffläche 11 ist ungleichmäßig durch eine Seitenfläche der Vertiefung ausgeformt, die konkav oder sogar in sich verdreht ausgeformt ist.

Innerhalb der Lauffläche 11 ist ein Rotor 30 angebracht, der beispielsweise einen elliptischen Umfang hat, so dass er das Schlauchsegment 20 bei der Rotation an seinen Hauptscheiteln 31, 32 leicht zusammendrücken kann. Hierzu sind am Rotor 30 nicht dargestellte Rollen vorgesehen, die am Schlauchsegment 20 abrollen und es dabei zusammendrücken. Durch die Drehung des Rotors 30 beispielsweise im Uhrzeigersinn bewegt sich der Bereich eines zusammengedrückten Schlauchsegments ebenfalls im Uhrzeigersinn, bis sich der zugehörige Hauptscheitel bzw. eine Rolle wieder vom Schlauchsegment löst. In der Zeit hat der gegenüber liegende Hauptscheitel jedoch bereits wieder Kontakt zu dem Schlauchsegment 20 aufgenommen, so dass Blut jeweils in dem Bereich des Schlauchsegments 20, vor dem es von dem Rotor 30 zusammendrückt wird, peristaltisch vom Pumpeneingang zum Pumpenausgang gefördert wird.

Der Rotor 30 dreht sich dabei um die Rotationsachse einer Antriebswelle der Pumpe, auf welche der Rotor 30 aufgesetzt ist. Dabei kann der Rotor 30 eine Aufnahme 33 aufweisen, mit welcher der Rotor 30 auf die Antriebswelle aufsteckbar ist. Ferner weist der Rotor 30 ein Verriegelungselement 50 zur axialen und drehfesten Verriegelung des Rotors 30 auf der Antriebswelle und zur Bereitstellung einer Kurbel für den manuellen Notbetrieb auf. In der in Fig. 2 dargestellten Ansicht ist das Verriegelungselement 50 dabei innerhalb der Aufnahme 33 versenkt.

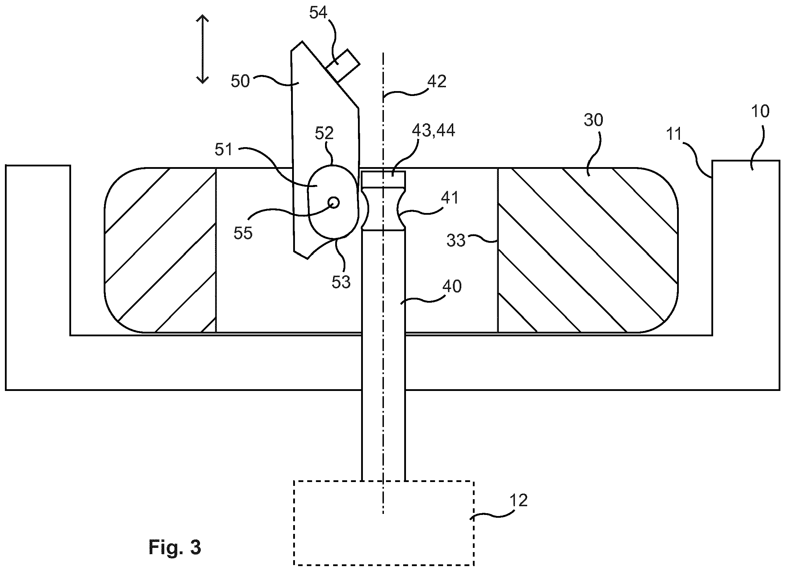

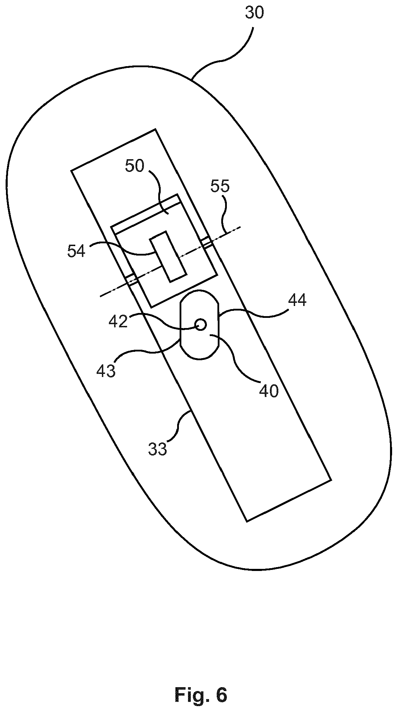

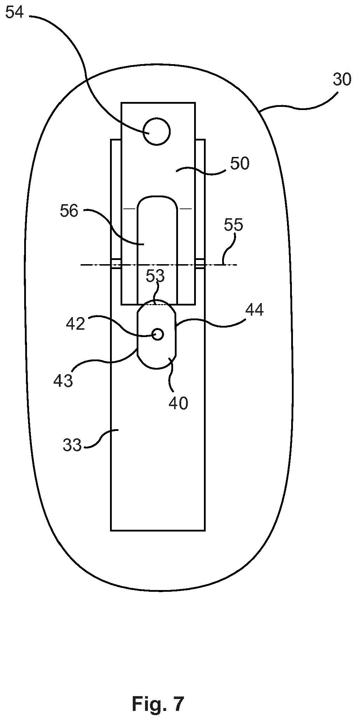

Fig. 3 zeigt eine schematische Seitenansicht eines Rotors 30 bei der Montage oder Demontage an der Antriebswelle 40 der Pumpe. Die Antriebswelle 40 bzw. Abtriebswelle der Pumpe wird von einem lediglich gestrichelt dargestellten Antrieb 12 angetrieben und rotiert um die Rotationsachse 42. Die Antriebswelle 40 weist dabei in ihrem Endbereich einen umlaufenden Radius 41 auf, so dass sie in diesem Bereich nach innen gewölbt ausgeformt ist. Oberhalb dieser nach innen gewölbten Fläche 41 ist die Antriebswelle 40 so ausgeformt, dass mit dem Verriegelungselement 50 ein Formschluss für eine drehfeste Verriegelung herstellbar ist. Beispielsweise sind dazu am Ende der Antriebswelle 40 zwei gegenüber liegende, ebene Seitenflächen 43 und 44 vorgesehen, wie sie auch den Aufsichten in den Figuren 6 und 7 zu entnehmen sind.

Ferner ist das Verriegelungselement 50 in der Nut 33 dargestellt, mit welcher der Rotor 30 auf die Antriebswelle 40 aufgesetzt werden kann. Das Verriegelungselement 50 umfasst zwei Seitenteile, von denen in Fig. 3 jedoch nur das hintere Seitenteil gezeigt ist. Zwischen diesen beiden Seitenteilen ist ein plattenförmiger Verriegelungsabschnitt 51 ausgeformt, so dass dieser Bereich 51 in Fig. 3 vor dem hinteren Seitenteil sichtbar ist. Dieser Verriegelungsabschnitt 51 ist so ausgeformt, dass er in den verschiedenen Stellungen des Verriegelungselements 50 die axiale Ver- und Entriegelung des Rotors 30 auf der Antriebswelle 40 übernimmt. Dazu weist der Verriegelungsabschnitt 51 zwei nach außen gewölbte Verriegelungsflächen 52 und 53 auf, die sich gegenüber liegen und durch zwei gerade Seitenflächen miteinander verbunden sind. Diese Seitenflächen können jedoch auch nach innen gewölbt sein. Die Außenwölbung der Verriegelungsflächen 52, 53 ist dabei jeweils so gewählt, dass sie sich für eine Verriegelung so in die Innenwölbung 41 der Antriebswelle 40 eindrehen kann, dass der Rotor 30 nicht axial von der Antriebswelle 40 gezogen werden kann.

Ferner ist das Verriegelungselement 50 gegenüber dem Rotor 30 um eine Drehachse 55 schwenkbar, die sich innerhalb des Verriegelungsbereichs 51 befindet. Die Aufhängung des Verriegelungselements 50 in der Nut 33 des Rotors 30 verläuft somit durch die beiden Seitenteile und den Verriegelungsabschnitt 51 des Verriegelungselements 50. Durch Schwenken des Verriegelungselements 50 um diese Drehachse 55 können die Verriegelungsflächen 52, 53 und die sie verbindenden geraden Seitenflächen in unterschiedliche Positionen bezüglich des umlaufenden Radius 41 der Antriebswelle 40 gebracht werden. In der in Fig. 3 dargestellten Position fügt sich keine der beiden Verriegelungsflächen 52, 53 in die Innenwölbung 41 der Antriebswelle 40 ein, sondern die verbindenden gerade Seitenflächen des Verriegelungsbereichs stehen in etwa parallel zur Antriebsachse 40. In dieser aufrechten Position des Verriegelungselements 50 findet keine axiale und keine drehfeste Verriegelung statt und der Rotor 30 kann entweder auf die Antriebswelle 40 aufgeschoben oder von dieser abgezogen werden, wie es durch den Doppelpfeil dargestellt ist. Diese Position wird für diese Erfindung auch als erste, vollständig entriegelte Lage des Verriegelungselements 50 bezeichnet.

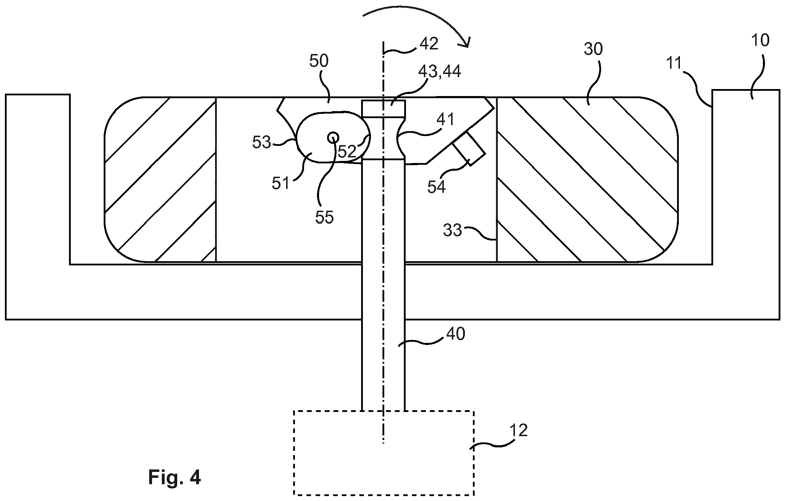

Durch Schwenken des Verriegelungselements 50 im Uhrzeigersinn um die Drehachse 55 gelangt das Element in eine erste axiale Verriegelungsposition, da nun die Verriegelungsfläche 52 in die Innenwölbung 41 der Antriebswelle 40 eingreift. Diese Stellung ist in Fig. 4 gezeigt. In dieser Position kann der Rotor 30 nicht nach oben von der Antriebswelle 40 abgezogen werden, da dies durch den oberen Rand des nach innen gewölbten Bereichs 41 der Antriebswelle 40 im Zusammenspiel mit dem Verriegelungsabschnitt 51 blockiert wird.

Gleichzeitig weist das Verriegelungselement 50 eine Geometrie auf, die beim Umlegen des Verriegelungselements 50 auf die Antriebswelle 40 mit dieser einen Formschluss in Drehrichtung erzeugt. Vorzugsweise weist das Verriegelungselement 50 dazu eine Nut 56 auf, an deren Innenflächen die gegenüber liegenden Seitenflächen 43 und 44 im oberen Bereich der Antriebswelle 40 anliegen und so einen Formschluss in Drehrichtung erzeugen. Diese Nut 56 kann beispielsweise durch die Seitenteile des Verriegelungselements 50 ausgeformt sein und ist der Fig. 7 in einer Aufsicht zu entnehmen.

Vorzugsweise greift der Formschluss in Drehrichtung zwischen dem Verriegelungselement 50 und der Antriebswelle 40, bevor der Verriegelungsabschnitt 51 den axialen Formschluss herstellt. Beim Umlegen des Verriegelungselements 50 auf die Antriebswelle gibt es somit einen Zwischenzustand, in dem der Rotor 30 bereits drehfest verriegelt ist, aber noch nicht axial. Beim weiteren Umlegen des Verriegelungselements 50 wird dann auch der axiale Formschluss hergestellt. In dieser Stellung des Verriegelungselements 50 kann die Schlauchrollenpumpe während der Therapie betrieben werden kann. Dabei ist das Verriegelungselement 50 vorzugsweise bündig in den Rotor 30 eingelassen, wobei ein Kurbelgriff 54 innerhalb der Nut 33 versenkt ist.

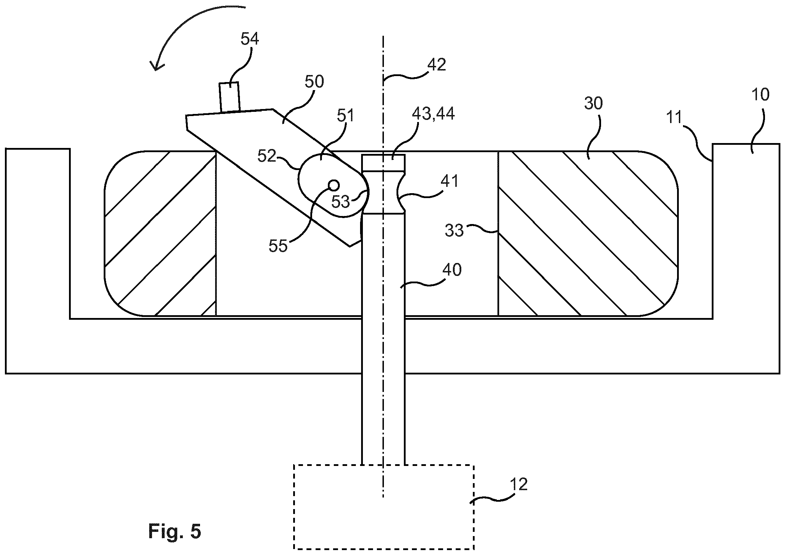

Durch Schwenken des Verriegelungselements 50 gegen den Uhrzeigersinn um die Drehachse 55 kann das Element über die mittlere, entriegelte Lage der Fig. 3 in eine zweite axiale Verriegelungsposition gelangen, da nun die Verriegelungsfläche 53 in die Innenwölbung 41 der Antriebswelle 40 eingreift. Diese Stellung ist in Fig. 5 gezeigt. Auch in dieser Position kann der Rotor 30 nicht nach oben von der Antriebswelle 40 abgezogen werden, da dies durch den oberen Rand der Antriebswelle 40 im Zusammenspiel mit dem Verriegelungsabschnitt 51 blockiert wird. In dieser Stellung des Verriegelungselements 50 kann die Pumpe somit ebenfalls während der Therapie betrieben werden. Allerdings ist in dieser Stellung der Formschluss in Drehrichtung aufgehoben, da die die Innenflächen der Nut 56 des Verriegelungselements 50 nicht mehr an der Antriebswelle 40 anliegt. In dieser Stellung befindet sich der Kurbelgriff 54 außerhalb des Grundkörpers des Rotors 30. Diese Kurbel kann von einem Bediener genutzt werden, um den Rotor 30 manuell um die Antriebswelle 40 zu drehen. Durch einen umlaufenden Radius 41 an der Antriebswelle 40 ist gewährleistet, dass der Rotor 30 bei der Drehung stets axial gegenüber der Antriebswelle 40 verriegelt ist.

Fig. 6 zeigt eine schematische Aufsicht auf einen Rotor 30 vor der Ausrichtung gegenüber einer Antriebswelle 40, wobei sich das Verriegelungselement 50 in der aufrechten, vollständig entriegelten Lage der Fig. 3 befindet. In dieser Lage ist keine axiale oder drehfeste Verriegelung vorhanden, so dass der Rotor 30 mit seiner Nut 33 auf die Antriebswelle 40 aufgesetzt und auch von dieser abgehoben werden kann. Ferner kann der Rotor 30 frei um die Antriebswelle 40 herum gedreht werden, bis die Innenflächen einer Nut innerhalb des Verriegelungselements 50 mit zwei gegenüber liegenden ebenen Seitenflächen 43 und 44 an der Antriebswelle 40 fluchten.

Diese Nut 56 im Verriegelungselement 50 ist in der Fig. 7 erkennbar, die eine Aufsicht auf den Rotor 30 in der dritten Verriegelungsposition der Fig. 5 darstellt, bei welcher der Kurbelgriff 54 für eine manuelle Betätigung oben aus dem Rotor 30 herausragt. Die Nut 56 ist beispielsweise zwischen den zwei Seitenteilen des Verriegelungselements 50 ausgebildet, zwischen denen sich auch der plattenförmige Verriegelungsabschnitt 51 für die axiale Verriegelung befindet. Dabei greift die nach außen gewölbte Verriegelungsfläche 53 des Verriegelungsbereichs 51 in die Innenwölbung der Antriebswelle 40 ein, was in Fig. 7 gestrichelt dargestellt ist.

In dieser Position und Ausrichtung des Verriegelungselements 50 fluchten die Innenseiten der Nut 56 mit den ebenen Seitenflächen der Antriebswelle 40, so dass eine drehfeste Verriegelung möglich wäre, wenn das Verriegelungselement 50 wieder in Richtung der Antriebswelle 40 umgelegt würde.

### Bezugszeichenliste:

- 10: Pumpengehäuse
- 11: Lauffläche
- 12: Antrieb
- 13: Arterieller Luftfänger
- 14: Venöser Luftfänger
- 15: Dialysator
- 16: Dialysat
- 17: Anzeige-/Eingabeeinheit, Touchscreen
- 18: Steuer-/Regelungseinheit
- 19: Medizinisches Gerät zur extrakorporalen Blutbehandlung, Dialysegerät
- 20: Schlauchsegment
- 21: Arterielles Blut vom Patienten
- 22: Venöses Blut zum Patienten
- 30: Rotor
- 31,32: Hauptscheitel
- 33: Aufnahme
- 40: Antriebswelle, Abtriebswelle der Pumpe
- 41: Umlaufender Radius, Innenwölbung (erster Verriegelungsabschnitt)
- 42: Rotationsachse der Antriebswelle
- 43,44: Ebene Seitenfläche der Antriebswelle (zweiter Verriegelungsabschnitt)
- 50: Verriegelungselement
- 51: Verriegelungsbereich (erster Verriegelungsabschnitt)
- 52, 53: Verriegelungsfläche
- 54: Kurbelgriff
- 55: Drehachse
- 56: Nut (zweiter Verriegelungsabschnitt)

## Patentansprüche

1. Schlauchrollenpumpe für ein medizinisches Gerät zur extrakorporalen Blutbehandlung mit einem Pumpengehäuse (10), das eine gebogene Lauffläche (11) und einen innerhalb der Lauffläche (11) drehbaren Rotor (30) aufweist, wobei ein Schlauchsegment (20) zwischen die Lauffläche (11) und den Rotor (30) einbringbar ist, und der Rotor (30) auf einer Antriebswelle (40) der Schlauchrollenpumpe anbringbar und sowohl in axialer Richtung als auch in Drehrichtung mit der Antriebswelle (30) koppelbar ist,
**dadurch gekennzeichnet, dass**
ein an dem Rotor (30) vorgesehenes Verriegelungselement (50) zwischen wenigstens zwei Lagen bewegbar, insbesondere schwenkbar, ist und die Geometrien der Antriebswelle (40) und des Verriegelungselements (50) so ausgeformt sind, dass der Rotor (30) in einer ersten Lage des Verriegelungselements (50) relativ zur Antriebswelle (40) verschiebbar und drehbar ist, während der Rotor (30) in einer zweiten Lage des Verriegelungselements (50) sowohl in axialer Richtung als auch in Drehrichtung mit der Antriebswelle (40), insbesondere durch Formschluss, gekoppelt ist.

2. Schlauchrollenpumpe nach Anspruch 1,
**dadurch gekennzeichnet, dass**
das Verriegelungselement (50) zumindest einen ersten Verriegelungsabschnitt (51) aufweist, der in der zweiten Lage zur axialen Verriegelung von Rotor (30) und Abtriebswelle (40) mit einem entsprechend an der Antriebswelle (40) ausgebildeten ersten Verriegelungsabschnitt (41) formschlüssig in Anlage kommt;
das Verriegelungselement (50) zumindest einen, insbesondere zum ersten Verriegelungsabschnitt (51) örtlich getrennten, zweiten Verriegelungsabschnitt (56) zur drehfesten Verriegelung (56) aufweist, der in der zweiten Lage zur drehfesten Verriegelung von Rotor (30) und Abtriebswelle (40) mit einem entsprechend an der Antriebswelle (40) ausgebildeten zweiten Verriegelungsabschnitt (43, 44) formschlüssig in Anlage kommt.

3. Schlauchrollenpumpe nach Anspruche 1 oder 2,
**dadurch gekennzeichnet, dass**
der erste Verriegelungsabschnitt (51) des Verriegelungselements (50) einen ersten, insbesondere runden, Vorsprung (52) aufweist,
der zweite Verriegelungsabschnitt der Antriebswelle (40) zumindest eine, insbesondere komplementär ausgebildete und umlaufende, Vertiefung (41) aufweist,
der erste Vorsprung (52) in der ersten Lage des Verriegelungselements (50) in die Vertiefung (41) eingreift.

4. Schlauchrollenpumpe nach einem der Ansprüche 1 bis 3,
**dadurch gekennzeichnet, dass**
der zweite Verriegelungsabschnitt des Verriegelungselements (50) eine Nut (56) aufweist,
der zweite Verriegelungsabschnitt der Antriebswelle (40) zumindest eine seitliche Abflachung (43, 44), insbesondere zwei auf einander gegenüber liegende Seiten ausgebildete Abflachungen (43, 44), aufweist, wobei
der zweite Verriegelungsabschnitt der Antriebswelle (40) in der zweiten Lage des Verriegelungselements (50) in die Nut (56) eingreift und die zumindest eine seitliche Abflachung (43, 44) der Antriebswelle (40) mit einer entsprechenden Innenseitenfläche der Nut (56) des Verriegelungselements (50) flächig in Anlage kommt.

5. Schlauchrollenpumpe nach einem oder mehreren der Ansprüche 2 bis 4,
**dadurch gekennzeichnet, dass** das Verriegelungselement (50) innerhalb einer Aufnahme (33) im Rotor (30) um eine senkrecht zur Drehachse (42) des Rotors (30) verlaufende und zu dieser beabstandete, insbesondere zentral durch den ersten Verriegelungsabschnitt verlaufende, Drehachse (55) schwenkbar ist.

6. Schlauchrollenpumpe nach einem oder mehreren der Ansprüche 1 bis 5,
**dadurch gekennzeichnet, dass** das Verriegelungselement (50) in eine dritte Lage bewegbar, insbesondere schwenkbar, ist, in welcher der Rotor (30) an der Antriebswelle (40) durch Formschluss axial verriegelt, jedoch drehbar ist.

7. Schlauchrollenpumpe nach Anspruch 6,
**dadurch gekennzeichnet, dass** der erste Verriegelungsabschnitt (51) einen, insbesondere runden, zweiten Vorsprung (53) aufweist, der in der dritten Lage des Verriegelungselements (50) in die Vertiefung (41) der Antriebswelle (40) eingreift.

8. Schlauchrollenpumpe nach Anspruch 7,
**dadurch gekennzeichnet, dass** sich die beiden Vorsprünge (52, 53) am erste Verriegelungsabschnitt (51) gegenüber liegen und durch zwei gerade Seitenflächen miteinander verbunden sind.

9. Schlauchrollenpumpe nach einem oder mehreren der Ansprüche 6 bis 8,
**dadurch gekennzeichnet, dass** die zweite und dritte Verriegelungslage durch Schwenken des Verriegelungselements (50) aus der ersten, vorzugsweise zur Drehachse (42) des Rotors parallelen, Lage in jeweils unterschiedliche Richtungen erreichbar sind.

10. Schlauchrollenpumpe nach einem oder mehreren der Ansprüche 6 bis 9,
**dadurch gekennzeichnet, dass** das Verriegelungselement (50) einen Kurbelgriff (54) aufweist, der in der dritten Lage des Verriegelungselements (50) aus der Aufnahme (33) im Rotor (30) herausragt.

11. Schlauchrollenpumpe nach einem oder mehreren der Ansprüche 1 bis 10,
**dadurch gekennzeichnet, dass** das Verriegelungselement (50) in der zweiten Lage des Verriegelungselements (50) bündig zur Außenkontur des Rotors (30) in die Aufnahme (33) eingelassen ist.

12. Schlauchrollenpumpe nach einem oder mehreren der Ansprüche 2 bis 11,
**dadurch gekennzeichnet, dass** der erste Verriegelungsabschnitt (51) plattenförmig zwischen zwei Seitenteilen des Verriegelungselements (50) ausgeformt ist.

13. Medizinisches Gerät zur extrakorporalen Blutbehandlung, umfassend eine Schlauchrollenpumpe mit einem Pumpengehäuse (10), das eine gebogene Lauffläche (11) und einen innerhalb der Lauffläche (11) drehbaren Rotor (30) aufweist, wobei ein Schlauchsegment (20) eines extrakorporalen Blutkreislaufs zwischen die Lauffläche (11) und den Rotor (30) einbringbar ist,
**dadurch gekennzeichnet, dass** die Schlauchrollenpumpe nach einem oder mehreren der Ansprüche 1 bis 12 ausgebildet ist.

## Claims

1. A tube roller pump for a medical device for extracorporeal blood treatment, comprising a pump housing (10) including a curved running surface (11) and a rotor (30) that is rotatable within the running surface (11), wherein a tube segment (20) is adapted to be placed between the running surface (11) and the rotor (30), and the rotor (30) is adapted to be attached to a drive shaft (40) of the tube roller pump and to be coupled to the drive shaft (40) in the axial direction as well as in the rotational direction,
**characterized in that**
a locking element (50) provided on the rotor (30) is movable, in particular pivotable, between at least two positions, and that the geometries of the drive shaft (40) and of the locking element (50) are configured such that, at a first position of the locking element (50), the rotor (30) is slidable and rotatable relative to the drive shaft (40), whereas, at a second position of the locking element (50), the rotor (30) is coupled to the drive shaft (40) in the axial direction as well as in the rotational direction, in particular through positive locking engagement with the drive shaft (40).

2. The tube roller pump according to claim 1,
**characterized in that**
the locking element (50) includes at least a first locking section (51), which, at the second position, is brought into positive locking contact with a complementary first locking section (41) formed on the drive shaft (40), so as to axially lock the rotor (30) and the drive shaft (40);
the locking element (50) comprises a second locking section (56) for torque-proof locking (56), said second locking section (56) being in particular locally separated from the first locking section (51) and being, at the second position, brought into positive locking contact with a complementary second locking section (43, 44) formed on the drive shaft (40), so as to lock the rotor (30) and the drive shaft (40) in a torque-proof manner.

3. The tube roller pump according to claim 1 or 2,
**characterized in that**
the first locking section (51) of the locking element (50) includes a first, in particular round, projection (52),
the second locking section of the drive shaft (40) includes at least one, in particular complementarily formed and circumferentially extending, recess (41),
the first projection (52) engages the recess (41) at the first position of the locking element (50).

4. The tube roller pump according to one of claims 1 to 3,
**characterized in that**
the second locking section of the locking element (50) includes a groove (56),
the second locking section of the drive shaft (40) includes at least one lateral flat area (43, 44), in particular two flat areas (43, 44) formed on opposed sides, wherein,
at the second position of the locking element (50), the second locking section of the drive shaft (40) engages the groove (56) and the at least one lateral flat area (43, 44) of the drive shaft (40) comes into large-area contact with a complementary inner side face of the groove (56) of the locking element (50).

5. The tube roller pump according to one or more of claims 2 to 4,
**characterized in that**
within a reception means (33) in the rotor (30), the locking element (50) is pivotable about an axis of rotation (55) extending in perpendicular, spaced relationship with the axis of rotation (42) of the rotor (30), in particular centrally through the first locking section.

6. The tube roller pump according to one or more of claims 1 to 5, **characterized in that**
the locking element (50) is adapted to be moved, in particular pivoted, to a third position in which the rotor (30) is axially locked on the drive shaft (40) by positive locking engagement therewith, but adapted to be rotated.

7. The tube roller pump according to claim 6,
**characterized in that**
the first locking section (51) includes a second projection (53), in particular a round projection (53), which, at the third position of the locking element (50), engages the recess (41) of the drive shaft (40).

8. The tube roller pump according to claim 7,
**characterized in that**
the two projections (52, 53) on the first locking section (51) are arranged in opposed relationship with one another and interconnected by two straight side faces.

9. The tube roller pump according to one or more of claims 6 to 8,
**characterized in that**
the second and third locking positions can be reached by pivoting the locking element (50) from the first position into respective different directions, said first position being preferably a position parallel to the axis of rotation (42) of the rotor.

10. The tube roller pump according to one or more of claims 6 to 9, **characterized in that**
the locking element (50) includes a crank handle (54) projecting beyond the reception means (33) in the rotor (30) at the third position of the locking element (50).

11. The tube roller pump according to one or more of claims 1 to 10, **characterized in that**
at the second position of the locking element (50), the locking element (50) is countersunk in the reception means (33) such that it is flush with the outer contour of the rotor (30).

12. The tube roller pump according to one or more of claims 2 to 11, **characterized in that**
the first locking section (51) is configured as a plate-shaped component extending between two side parts of the locking element (50).

13. A medical device for extracorporeal blood treatment, comprising a tube roller pump including a pump housing (10) having a curved running surface (11) and a rotor (30) that is rotatable within the running surface (11), a tube segment (20) of an extracorporeal blood circuit being adapted to be placed between the running surface (11) and the rotor (30),
**characterized in that**
the tube roller pump is configured according to anyone of the claims 1 to 12.

## Revendications

1. Pompe à rouleau tubulaire pour un appareil médical pour le traitement extracorporel du sang avec un boîtier de pompe (10), qui présente une surface de roulement courbe (11) et un rotor (30) rotatif à l'intérieur de la surface de roulement (11), dans laquelle un segment tubulaire (20) peut être inséré entre la surface de roulement (11) et le rotor (30), et le rotor (30) peut être monté sur un arbre d'entraînement (40) de la pompe à rouleau tubulaire, et peut être couplé aussi bien dans la direction axiale que dans le sens de rotation avec l'arbre d'entraînement (30), **caractérisée en ce que**
un élément de verrouillage (50) prévu au niveau du rotor (30) est mobile, en particulier pivotant, entre au moins deux positions et les géométries de l'arbre d'entraînement (40) et de l'élément de verrouillage (50) sont formées de sorte que le rotor (30) est coulissant et rotatif dans une première position de l'élément de verrouillage (50) par rapport à l'arbre d'entraînement (40), pendant que le rotor (30) est couplé, en particulier par correspondance de forme, dans une deuxième position de l'élément de verrouillage (50) aussi bien dans la direction axiale que dans le sens de rotation avec l'arbre d'entraînement (40).

2. Pompe à rouleau tubulaire selon la revendication 1,
**caractérisée en ce que**
l'élément de verrouillage (50) présente au moins une première section de verrouillage (51), qui vient en appui par correspondance de forme dans la deuxième position pour le verrouillage axial du rotor (30) et de l'arbre de sortie (40) avec une première section de verrouillage (41) réalisée en conséquence au niveau de l'arbre d'entraînement (40) ;
l'élément de verrouillage (50) présente au moins une deuxième section de verrouillage (56), en particulier séparée localement de la première section de verrouillage (51), pour le verrouillage solidaire en rotation (56), qui vient en appui par correspondance de forme dans la deuxième position pour le verrouillage solidaire en rotation du rotor (30) et de l'arbre de sortie (40) avec une deuxième section de verrouillage (43, 44) réalisée de manière correspondante au niveau de l'arbre d'entraînement (40).

3. Pompe à rouleau tubulaire selon la revendication 1 ou 2,
**caractérisée en ce que**
la première section de verrouillage (51) de l'élément de verrouillage (50) présente une première saillie (52), en particulier circulaire,
la deuxième section de verrouillage de l'arbre d'entraînement (40) présente au moins un évidement (41), en particulier périphérique et réalisé de manière complémentaire, la première saillie (52) entre en prise dans l'évidement (41) dans la première position de l'élément de verrouillage (50).

4. Pompe à rouleau tubulaire selon l'une quelconque des revendications 1 à 3, **caractérisée en ce que**
la deuxième section de verrouillage de l'élément de verrouillage (50) présente une rainure (56),
la deuxième section de verrouillage de l'arbre d'entraînement (40) présente au moins un aplatissement latéral (43, 44), en particulier deux aplatissements (43, 44) réalisés sur des côtés opposés l'un à l'autre, dans laquelle
la deuxième section de verrouillage de l'arbre d'entraînement (40) entre en prise dans la rainure (56) dans la deuxième position de l'élément de verrouillage (50) et l'au moins un aplatissement latéral (43, 44) de l'arbre d'entraînement (40) vient en appui plat avec une surface latérale intérieure correspondante de la rainure (56) de l'élément de verrouillage (50).

5. Pompe à rouleau tubulaire selon une ou plusieurs des revendications 2 à 4, **caractérisée en ce que** l'élément de verrouillage (50) peut pivoter à l'intérieur d'un logement (33) dans le rotor (30) autour d'un axe de rotation (55) s'étendant perpendiculairement à l'axe de rotation (42) du rotor (30) et s'étendant à distance de celui-ci, en particulier de manière centrale par la première section de verrouillage.

6. Pompe à rouleau tubulaire selon une ou plusieurs des revendications 1 à 5, **caractérisée en ce que** l'élément de verrouillage (50) est mobile, en particulier pivotant, dans une troisième position, dans laquelle le rotor (30) est verrouillé axialement par correspondance de forme au niveau de l'arbre d'entraînement (40), mais est tout de même rotatif.

7. Pompe à rouleau tubulaire selon la revendication 6,
**caractérisée en ce que** la première section de verrouillage (51) présente une deuxième saillie (53), en particulier circulaire, qui vient en prise dans l'évidement (41) de l'arbre d'entraînement (40) dans la troisième position de l'élément de verrouillage (50).

8. Pompe à rouleau tubulaire selon la revendication 7,
**caractérisée en ce que** les deux saillies (52, 53) sont opposées au niveau de la première section de verrouillage (51) et sont reliées l'une à l'autre par deux surfaces latérales rectilignes.

9. Pompe à rouleau tubulaire selon une ou plusieurs des revendications 6 à 8, **caractérisée en ce que** la deuxième et la troisième position de verrouillage sont accessibles par pivotement de l'élément de verrouillage (50) de la première position, de préférence parallèle à l'axe de rotation (42) du rotor, dans des directions respectivement différentes.

10. Pompe à rouleau tubulaire selon une ou plusieurs des revendications 6 à 9, **caractérisée en ce que** l'élément de verrouillage (50) présente une poignée de manivelle (54), qui fait saillie du logement (33) dans le rotor (30) dans la troisième position de l'élément de verrouillage (50).

11. Pompe à rouleau tubulaire selon une ou plusieurs des revendications 1 à 10, **caractérisée en ce que** l'élément de verrouillage (50) est encastré dans la deuxième position de l'élément de verrouillage (50) à fleur par rapport au contour extérieur du rotor (30) dans le logement (33).

12. Pompe à rouleau tubulaire selon une ou plusieurs des revendications 2 à 11, **caractérisée en ce que** la première section de verrouillage (51) est réalisée en forme de plaque entre deux parties latérales de l'élément de verrouillage (50).

13. Appareil médical pour le traitement extracorporel du sang, comprenant une pompe à rouleau tubulaire avec un boîtier de pompe (10), qui présente une surface de roulement courbe (11) et un rotor (30) rotatif à l'intérieur de la surface de roulement (11), dans lequel un segment tubulaire (20) d'une circulation extracorporelle du sang peut être inséré entre la surface de roulement (11) et le rotor (30),
**caractérisé en ce que** la pompe à rouleau tubulaire est réalisée selon une ou plusieurs des revendications 1 à 12.
